# EUROPÄISCHE PATENTANMELDUNG

(11) **EP 3 424 528 A1**
(43) Veröffentlichungstag der Anmeldung: **09.01.2019**
(21) Anmeldenummer: 18162672.2
(22) Anmeldetag: 19.03.2018
(51) Int. Cl.: A61K 39/395, A61P 35/02

(54) **BIOLOGISCHE BINDEMOLEKÜLE**

(30) Priorität: 07.07.2017 EP 17001166; 07.07.2017 EP 17001167
(71) Anmelder: AVA Lifescience GmbH, 79211 Denzlingen (DE)
(72) Erfinder: KLAPPROTH, Holger, 79018 Freiburg i. Breisgau (DE); BIRSNER, Ulrich, 79110 Freiburg i. Breisgau (DE); KESSEMEIER, Marc A., 79312 Emmendingen (DE); JUMAA, Hassan, 89143 Blaubeuren (DE)
(74) Vertreter: Stürken, Joachim

(57) **Zusammenfassung**

Die vorliegende Erfindung betrifft das Gebiet der Herstellung, Identifizierung und Selektion von biologischen Bindemolekülen wie Antikörpern oder Fragmenten derselben, sowie deren Verwendung im Rahmen der Therapie und Prophylaxe von Krebserkrankungen wie insbesondere malignen B-Zell Neoplasien.

Die Bindemoleküle sind in der Lage, selektiv an autonom aktive oder autonom aktivierte B-Zell Rezeptoren zu binden, wobei die autonom aktiven oder autonom aktivierten B-Zell Rezeptoren durch das Vorhandensein von strukturellen Domänen charakterisiert sind, an die das Bindemolekül selektiv bindet und die für den autonom aktiven oder autonom aktivierten Zustand der B-Zell Rezeptoren ursächlich sind.

SEQUENCE LISTING

<110> Klapproth Holger
      Birnser Ulrich
      Kessemeier Marc
      Jumaa Hassan

<120> Therapeutischer Antikörper zur Therapie der CLL

<130> CLL-Epitop

<160> 16

<170> BiSSAP 1.3.6

<210> 1
<211> 10
<212> PRT
<213> Homo sapiens


<220>
<223> Autonom aktiver Bereich Subset 2/1

<400> 1
Lys Leu Thr Val Leu Arg Gln Pro Lys Ala
1               5                   10

<210> 2
<211> 8
<212> PRT
<213> Homo sapiens


<220>
<223> Autonom aktiver Bereich Subset 2/2

<400> 2
Val Ala Pro Gly Lys Thr Ala Arg
1               5

<210> 3
<211> 10
<212> PRT
<213> Homo sapiens

<220>
<223> Autonom aktiver Bereich Subset 4/1

<400> 3
Pro Thr Ile Arg Arg Tyr Tyr Tyr Tyr Gly
1               5                   10

<210> 4
<211> 9
<212> PRT
<213> Homo sapiens


<220>
<223> Autonom aktiver Bereich Subset 4/2

<400> 4
Asn His Lys Pro Ser Asn Thr Lys Val
1               5

<210> 5
<211> 116
<212> PRT

<213> Homo sapiens

<220>
<223> Autonom aktiver Bereich Subset 2 VH

<400> 5
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Arg Ser Tyr
            20                  25                  30
Ser Met Asn Trp Val Arg Gln Ala Pro Gly Lys Gly Leu Glu Trp Val
        35                  40                  45
Ser Ser Ile Ile Ser Ser Ser Ser Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr Tyr Cys
                85                  90                  95
Ala Arg Asp Gln Asn Ala Met Asp Val Trp Gly Gln Gly Thr Thr Val
            100                 105                 110
Thr Val Ser Ser
        115

<210> 6
<211> 215
<212> PRT
<213> Homo sapiens

<220>
<223> Autonom aktiver Bereich Subset 2 LC

<400> 6
Ser Tyr Glu Leu Thr Gln Pro Pro Ser Val Ser Val Ala Pro Gly Lys
1               5                   10                  15
Thr Ala Arg Ile Thr Cys Ala Gly Asn Asn Ile Gly Ser Lys Ser Val
            20                  25                  30
His Trp Tyr Gln Gln Lys Pro Gly Gln Ala Pro Val Leu Val Ile Tyr
        35                  40                  45
Tyr Asp Ser Asp Arg Pro Ser Gly Ile Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Gln Val Trp Asp Ser Gly Ser Asp His
                85                  90                  95
Pro Trp Val Phe Gly Gly Gly Thr Lys Leu Thr Val Leu Arg Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215

<210> 7
<211> 224
<212> PRT
<213> Homo sapiens

<220>
<223> Autonom aktiver Bereich Subset 4 HC

<400> 7
Gln Val Gln Leu Gln Gln Trp Gly Ala Gly Leu Leu Lys Pro Ser Glu
1               5                   10                  15
Thr Leu Ser Leu Thr Cys Ala Val Tyr Gly Gly Ser Phe Ser Gly Tyr
            20                  25                  30
Tyr Trp Thr Trp Ile Arg Gln Ser Pro Gly Lys Gly Leu Glu Trp Ile
        35                  40                  45
Gly Glu Ile Asn His Ser Gly Ser Thr Thr Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Asn Ser Val Thr Ala Ala Asp Thr Ala Val Tyr Tyr Cys Ala
                85                  90                  95
Arg Gly Tyr Gly Asp Thr Pro Thr Ile Arg Arg Tyr Tyr Tyr Tyr Gly
            100                 105                 110
Met Asp Val Trp Gly Gln Gly Thr Thr Val Thr Val Ser Ser Ala Ser
        115                 120                 125
Thr Lys Gly Pro Ser Val Phe Pro Leu Ala Pro Ser Ser Lys Ser Thr
    130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
                165                 170                 175
His Thr Phe Pro Ala Cys Leu Gln Ser Ser Gly Leu Tyr Ser Leu Ser
            180                 185                 190
Ser Val Val Thr Val Pro Ser Ser Ser Leu Gly Thr Gln Thr Tyr Ile
        195                 200                 205
Cys Asn Val Asn His Lys Pro Ser Asn Thr Lys Val Asp Lys Lys Cys
    210                 215                 220

<210> 8
<211> 220
<212> PRT
<213> Homo sapiens


<220>
<223> Autonom aktiver Bereich Subset 4 LC

<400> 8
Asp Ile Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val His Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asp Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Leu Tyr Tyr Cys Met Gln Gly
                85                  90                  95
Thr His Trp Pro Pro Tyr Thr Phe Gly Gln Gly Thr Lys Val Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr
            180                 185                 190
Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210> 9
<211> 119
<212> PRT
<213> Mus musculus

<220>
<223> AVA mAb01 HC

<400> 9
Gln Val Gln Leu Gln Gln Ser Gly Pro Gly Leu Val Gln Pro Ser Gln
1               5                   10                  15
Ser Leu Ser Ile Thr Cys Thr Val Ser Gly Phe Ser Leu Thr Ser Tyr
            20                  25                  30
Gly Ile His Trp Val Arg Gln Ser Pro Gly Lys Gly Leu Glu Trp Leu
        35                  40                  45
Gly Val Ile Trp Arg Gly Gly Gly Thr Asp Ser Asn Ala Ala Phe Met
    50                  55                  60
Ser Arg Leu Ser Ile Thr Lys Asp Asn Ser Lys Ser Gln Val Phe Phe
65                  70                  75                  80
Lys Met Asn Ser Leu Gln Ala Asp Asp Thr Ala Ile Tyr Tyr Cys Ala
                85                  90                  95
Arg Ser Arg Tyr Asp Glu Glu Glu Ser Met Asn Tyr Trp Gly Gln Gly
            100                 105                 110
Thr Ser Val Thr Val Ser Ser
        115

<210> 10
<211> 107
<212> PRT
<213> Mus musculus

<220>
<223> AVA mAb01 LC

<400> 10
Gln Ile Val Leu Thr Gln Ser Pro Ala Ser Leu Ser Ala Ser Val Gly
1               5                   10                  15
Glu Thr Val Thr Ile Thr Cys Arg Ala Ser Gly Asn Ile His Ser Tyr
            20                  25                  30
Leu Ala Trp Tyr Gln Gln Lys Gln Gly Lys Ser Pro Gln Leu Leu Val
        35                  40                  45
Tyr Asn Ala Lys Thr Leu Ala Asp Gly Val Pro Ser Arg Phe Ser Gly
    50                  55                  60
Ser Gly Ser Gly Thr Gln Tyr Ser Leu Lys Ile Asn Ser Leu Gln Pro
65                  70                  75                  80
Glu Asp Phe Gly Ser Tyr Tyr Cys Gln His Phe Trp Asn Thr Pro Pro
                85                  90                  95
Thr Phe Gly Ala Gly Thr Lys Leu Glu Leu Lys
            100                 105

<210> 11
<211> 8
<212> PRT
<213> Mus musculus

<220>
<223> AVA mAb01 CDR1 HC

<400> 11
Gly Phe Ser Leu Thr Ser Tyr Gly
1               5

<210> 12

<211> 7
<212> PRT
<213> Mus musculus

<220>
<223> AVA mAb01 CDR2 HC

<400> 12
Ile Trp Arg Gly Gly Gly Thr
1               5

<210> 13
<211> 13
<212> PRT
<213> Mus musculus

<220>
<223> AVA mAb01 CDR3 HC

<400> 13
Ala Arg Ser Arg Tyr Asp Glu Glu Glu Ser Met Asn Tyr
1               5                   10

<210> 14
<211> 6
<212> PRT
<213> Mus musculus

<220>
<223> AVA mAb01 CDR1 LC

<400> 14
Gly Asn Ile His Ser Tyr
1               5

<210> 15
<211> 4
<212> PRT
<213> Mus musculus

<220>
<223> AVA mAb01 CDR2 LC

<400> 15
Asn Ala Lys Thr
1

<210> 16
<211> 9
<212> PRT
<213> Mus musculus

<220>
<223> AVA mAb01 CDR3 LC

<400> 16
Gln His Phe Trp Asn Thr Pro Pro Thr
1               5

<210> 17
<211> 8
<212> PRT
<213> Mus musculus

<220>
<223> Autonom aktiver Bereich Subset 4/2

<400> 17
Val Ser Ser Ala Ser Thr Lys Gly
1               5

## Beschreibung

Die vorliegende Erfindung betrifft das Gebiet der Herstellung, Identifizierung und Selektion von Antikörpern oder Fragmenten derselben, sowie deren Verwendung im Rahmen der Prophylaxe und Therapie von Krebserkrankungen wie insbesondere malignen B-Zell Neoplasien.

Mit malignen B-Zell Neoplasien werden allgemein maligne Erkrankungen des blutbildenden oder des lymphatischen Systems bezeichnet. Sie umfassen Krankheitsbilder wie beispielsweise die Leukämie und zählen im weiteren Sinne zu den Krebserkrankungen. Leukämien zeichnen sich durch eine stark vermehrte Bildung von funktionsuntüchtigen Vorläuferzellen der weißen Blutzellen aus, welche auch Leukämiezellen genannt werden. Diese Zellen breiten sich im Knochenmark aus, verdrängen dort die übliche Blutbildung und akkumulieren in der Regel auch stark vermehrt im peripheren Blut. Sie können Leber, Milz, Lymphknoten und weitere Organe infiltrieren und dadurch deren Funktion beeinträchtigen. Die Störung der Blutbildung führt zu einer Verminderung der normalen Blutbestandteile, wodurch eine Anämie durch Mangel an Sauerstoff transportierenden roten Blutkörperchen, ein Mangel an blutungsstillenden Blutplättchen, und ein Mangel an reifen funktionstüchtigen weißen Blutzellen entstehen können.

Je nach Krankheitsverlauf unterscheidet man zwischen akuter und chronischer Leukämie. Akute Leukämien sind lebensbedrohliche Erkrankungen, die unbehandelt in wenigen Wochen bis Monaten zum Tode führen. Chronische Leukämien hingegen verlaufen meist über mehrere Jahre und sind im Anfangsstadium häufig symptomarm.

Die wichtigsten Leukämieformen sind:
- akute myeloische Leukämie (AML)
- chronische myeloische Leukämie (CML)
- akute lymphatische Leukämie (ALL)
- chronische lymphatische Leukämie (CLL).

Üblicherweise werden Leukämien im Rahmen einer Chemotherapie behandelt. Neuere Therapieformen setzen dabei vermehrt auf die Verwendung von monoklonalen Antikörpern wie z.B. GA101 (Obinutuzumab), der ähnlich wie Rituximab und Ofatumumab als CD20-Antikörper wirkt und zur Behandlung der chronischen lymphatischen Leukämie (CLL) eingesetzt wird. Durch Verwendung dieser Antikörper kann die remissionsfreie Zeit um ca. 10 Monate verlängert werden.

Weitere maligne Erkrankungen des blutbildenden oder des lymphatischen Systems (maligne B-Zell Neoplasien) betreffen Lymphome, wie z.B. das Hodgkin Lymphom und die B-Zell Varianten der Non-Hodgkin Lymphome.

Werden Antikörper gegen Rezeptoren generiert, so werden in der Regel Tiere mit dem Rezeptor (aufgereinigt, kloniert, oder als Peptidfragmente) immunisiert und Hybridomazellen erzeugt. Diese Hybridomazellen produzieren Antikörper, die dann mittels ELISA oder mittels exprimierter Rezeptoren in Zellsystemen getestet werden. Dazu werden herkömmlich etablierte Zelllinien verwendet, da sich nur diese einfach kultivieren lassen. Dabei können Antikörper erzeugt werden, die relativ spezifisch an einen bestimmten Rezeptortyp (z.B. Anti-IgGl, Anti-IgE) binden. Hierbei kommt es jedoch häufig zu Kreuzreaktionen mit anderen Rezeptoren oder anderen Epitopen.

Für eine therapeutische Anwendung von BCR-Antikörpern ist es zumeist nicht ausreichend, nur einen Antikörper gegen den BCR im allgemeinen einzusetzen, da ein solcher Breitbandeinsatz erhebliche Nebenwirkungen auslösen kann. Vielmehr wäre es wünschenswert, einen Antikörper bereitzustellen, der selektiv an einen Rezeptor bindet, der eine (pathophysiologische) Aktivierung wie insbesondere eine autonome Aktivierung aufweist. Ein solcher Antikörper ist im Stand der Technik nicht bekannt und ein Verfahren zu dessen Herstellung oder Gewinnung durch Selektion existiert nicht.

Die Therapien des Standes der Technik zur Behandlung von Leukämien sind für den Patienten daher zumeist sehr belastend. Generell kann man zusammenfassen, dass die unerwünschten Nebenwirkungen der Therapie und die oftmals nicht ausreichende Wirkung der Medikamente zu einer hohen Todesrate dieser Erkrankung führen, denn es werden nicht nur Tumorzellen, sondern auch gesunde Zellen des Immunsystems geschädigt. Zudem kommt es oftmals nicht zu einer Heilung, sondern nur zur Schaffung eines gewissen Zeitraums, in welchem die Erkrankung demissionsfrei verläuft.

Die Aufgabe der vorliegenden Erfindung liegt daher in der Bereitstellung alternativer Konzepte und Wirkstoffe wie insbesondere alternativer Antikörper zur prophylaktischen und/oder therapeutischen Verwendung, mit denen die bestehenden Probleme des Standes der Technik überwunden werden.

Bevor auf die einzelnen Aspekte der vorliegenden Erfindung detailliert eingegangen wird, erfolgt eine Klärung relevanter Begriffe, die im Rahmen der vorliegenden Beschreibung verwendet werden.

Der vorliegend verwendete Begriff "Neoplasie" bezeichnet generell eine Neubildung von Körpergewebe. Handelt es sich dabei um eine krankhafte oder bösartige Erscheinungsform, spricht man von einer malignen Neoplasie. Bei einer malignen B-Zell Neoplasie handelt es sich also um eine bösartige und unkontrollierte Neugewebsbildung von B-Zellen, wobei diese Begrifflichkeit alle B-Zell assoziierten Krebserkrankungen wie z.B. Leukämien und B-Zell Lymphome gleichermaßen betrifft.

Ein "Bereich zum Abtöten von Neoplasien" kann Neoplasien entweder als Folge einer direkten oder indirekten Wirkung abtöten. Im Falle der therapeutischen Verwendung spezifischer Antikörper wird ein Molekül an den Antikörper oder an ein funktionelles Fragment dieses Antikörpers oder eines anderen, diesen Antikörper oder sein Fragment umfassendes biologisches Bindemolekül gebunden, um so eine Wirkung auszuüben, die über die Wirkung der Bindung des Antikörpers oder seines Fragments hinausgeht. Ein solches Molekül kann beispielsweise aus der Gruppe bestehend aus einem Immuntoxin, einem Zytokin, einem Chelator, einem Radioisotop, und Kombinationen davon ausgewählt sein.

Unter "biologischen Bindemolekülen" werden vorliegend beispielsweise, aber nicht ausschließlich, Antikörper inklusive Fusionsproteinen verstanden. Vorteilshafterweise, und daher bevorzugt, ist ein solcher Antikörper ausgewählt aus der Gruppe bestehend aus einem IgG Antikörper, einem IgM Antikörper, einem humanisierten IgG Antikörper, und einem humanen Antikörper, in den die Erkennungssequenz des Epitops eingefügt ist. Ein derartiges Bindemolekül kann auch in Form eines funktionellen Fragments des gesamten Antikörpers bereitgestellt sein, z.B. als Fab-Fragment. Ein Bindemolekül kann auch noch weitere Bereiche umfassen, die z.B. zum Abtöten/Absterben von Neoplasien führen und demgemäß die Funktionalität eines Immuntoxins und/oder Immunzytokins aufweisen. Insbesondere kann ein solches Bindemolekül auch membran- oder zellständig sein. Eine solche membranständige Form eines Bindemoleküls ist z.B. der chimäre Antigenrezeptor ("Chimeric-Antigen Receptor"; CAR) auf CAR-T Zellen.

Die Aufgabe des B-Zell Rezeptors oder B-Zell Rezeptorkomplexes (BCR) auf der Oberfläche einer B-Zelle ist es, Pathogene zu erkennen und zu binden, weshalb er quasi als membrangebundener Antikörper angesehen werden kann. Diese Bindung führt zu einer Konformationsänderung des BCRs, wodurch eine Signalkaskade ausgelöst wird, die letzten Endes zu einer Aktivierung der B-Zelle führt. Der BCR wird in großer Vielfalt in reifenden B-Zellen gebildet.

Die Entwicklung der B-Zellen findet beim Menschen und auch bei einigen anderen Säugern im Knochenmark bzw. in der fetalen Leber statt. Die Signale, die für das Entwicklungsprogramm notwendig sind, erhalten die sich entwickelnden Lymphozyten von so genannten Stromazellen. Bei der B-Zell-Entwicklung ist die Bildung eines funktionierenden B-Zell Rezeptors (die membrangebundene Form des 'Antikörpers') von entscheidender Bedeutung. Nur mit diesem Antigenrezeptor sind reife B-Zellen später in der Lage, fremde Antigene zu erkennen und durch die Bildung von entsprechenden Antikörpern an feindliche Strukturen zu binden. Die Antigenspezifität des Rezeptors wird durch die Verknüpfung bestimmter Gensegmente bestimmt. Die Segmente heißen V-, D- und J-Segmente, weshalb der Prozess als V(D)J-Rekombination bezeichnet wird. Dabei werden diese Segmente, die den Antigen-bindenden Teil des B-Zell-Rezeptors bilden, umgeordnet. Der gesamte Rezeptor besteht aus zwei identischen leichten Proteinketten und zwei identischen schweren Proteinketten, wobei die schweren mit den leichten Ketten jeweils über Disulfidbrücken miteinander verknüpft sind. Bei der VDJ-Rekombination werden zuerst die V-, D- und J-Segmente der schweren Kette des B-Zell Rezeptors verknüpft, danach die V- und J-Segmente der leichten Rezeptorkette. Nur wenn die Gene dabei erfolgreich umgeordnet werden, was als produktive Genumordnung bezeichnet wird, kann die Zelle in den jeweils nächsten Entwicklungsschritt übergehen.

B-Zellen, die während ihrer Ausreifung im Knochenmark auf körpereigene Antigene reagieren, sterben in den allermeisten Fällen durch Apoptose ab. Im Blut von gesunden Menschen lassen sich geringe Mengen an autoreaktiven Zellen, unter anderem gegen Thyreoglobulin oder auch Kollagen, nachweisen (Abul K. Abbas: Diseases of Immunity in Vinay Kumar, Abul K. Abbas, Nelson Fausto : Robbins and Cotran - Pathologic Basis of Disease. 7. Auflage. Philadelphia 2005, S. 224f).

Da der Prozess der Generierung eines solchen BCRs auf einer zufälligen Zusammenlagerung von Gensegmenten basiert, kann es vorkommen, dass der neu entstandene BCR unerwünscht körpereigene Strukturen erkennt und so "daueraktiviert" wird. Um die Entstehung eines derart "dauerhaft aktiven oder aktivierten" BCRs zu verhindern, existieren verschiedene körpereigne Schutzmechanismen. Werden diese allerdings auf Grund einer krankhaften Veränderung der sich entwickelnden B-Zelle überwunden, kann sich daraus eine maligne oder auch autoimmun manifestierende Erkrankung entwickeln.

Bei einem "autonom aktiven" oder "autonom aktivierten" BCR handelt es sich demgegenüber um eine besondere Art eines dauerhaft aktiven BCRs. Während die konventionelle Aktivierung von einem externen Antigen ausgeht (s.o.), resultiert der autonom aktive BCR aus seiner Interaktion mit Membranstrukturen auf der Oberfläche derselben Zelle. Für das Krankheitsbild der CLL konnte eine die autonome Aktivierung auslösende Interaktion zwischen BCRs gezeigt werden, die sich zueinander benachbart auf der Oberflache derselben Zelle befanden (M. Dühren-von Minden et. al; Nature 2012). Ein weiteres Beispiel für einen autonom aktiven BCR stellt der pre-BCR dar, der im Laufe der Entwicklung einer B-Zelle als Entwicklungscheck exprimiert wird. Neben der Interaktion benachbarter Rezeptoren (BCR:BCR) kann aber auch eine Interaktion zwischen Rezeptor und einem Membranprotein (BCR:Membranprotein) zu einem autonom aktiven oder aktivierten BCR führen.

Die erfindungsgemäße Lösung dieser Probleme beruht auf der überraschenden Erkenntnis, dass sich auf Tumorzellen von Patenten mit CLL B-Zell Rezeptoren befinden, die autonom aktiv oder autonom aktiviert sind, und dass diese autonom aktiven oder aktivierten Rezeptoren durch das Vorhandensein gemeinsamer Epitope, die in entsprechenden Rezeptoren von gesunden Zellen desselben Patienten nicht nachgewiesen werden können, gekennzeichnet sind. Diese Zellen können somit aufgrund der Anwesenheit von autonom aktiven B-Zell Rezeptoren, die durch das Vorkommen der oben genannten Epitope gekennzeichnet sind, durch einen Antikörper spezifisch erkannt und therapiert werden, sodass gesunde B-Zellen ohne diese Charakteristik dadurch nicht in Mitleidenschaft gezogen werden, wodurch die Behandlung deutlich spezifischer und mit weniger unerwünschten Nebenwirkungen durchgeführt werden kann.

Im Rahmen der zahlreichen für die vorliegende Erfindung durchgeführten Experimente stellte sich jedoch überraschenderweise heraus, dass Antikörper mit besonderer Spezifität für diese modifizierten Rezeptorbereiche (Epitope) nicht mit üblichen Standardmethoden hergestellt und selektioniert werden können. Erst nachdem die experimentellen Bedingungen derart angepasst wurden, dass im Rahmen von Bindungsstudien gentechnisch veränderte Zellen eingesetzt wurden, deren modifizierte B-Zell Rezeptoren sich in einem nativen und aktivierten Zustand befanden, konnten geeignete Antikörper mit der gewünschten und erforderlichen Spezifität gewonnen werden. Mit anderen Worten ist es für die erfindungsgemäß vorgeschlagenen Lösungen von essenzieller Bedeutung, dass die in Bindungsstudien zur Selektion geeigneter prophylaktischer oder therapeutischer Antikörper eingesetzten Zellen ihre modifizierten Bereiche (Epitope) in weitgehend nativer und aktivierter Form präsentieren. Hierbei zeigte es sich, dass sog. pro-/prä-B-Zellen aufgrund ihrer physiologischen Konstitution besonders geeignet sind. Die Bereitstellung derartig spezifischer Antikörper und funktioneller Fragmente derselben, die ebenfalls über dieses spezifische Bindungsverhalten verfügen, ermöglicht somit eine Tumorspezifische Behandlung, die durch einen deutlich verbesserten Behandlungserfolg und, dank der Reduktion von unerwünschten systemischen Wirkungen, einen deutlich erhöhten Therapieerfolg gekennzeichnet ist.

Wie bereits erwähnt, werden durch die vorliegende Erfindung biologische Bindemoleküle in Form von Antikörpern oder funktionellen Fragmenten derselben und ein Verfahren zur Herstellung (Identifizierung und Selektion) solcher Bindemoleküle bereitgestellt, welche selektiv an die modifizierten Epitope autonom aktiver membranständiger Immunglobuline von B-Zell Neoplasien binden. Nach einer bevorzugten Ausführungsform der Erfindung binden die biologischen Bindeproteine selektiv an solche autonom aktive B-Zell Rezeptoren auf B-Zellen, die bei immunologischen (z.B. Autoimmun-) Erkrankungen auftreten und mit diesen in ursächlichem Zusammenhang stehen (z.B. Allergien, Colitis ulcerosa, Diabetes mellitus Typ 1, Multiple Sklerose, Psoriasis, Rheumatisches Fieber, Rheumatoide Arthritis, Zöliakie). Ferner werden sowohl prophylaktische als auch therapeutische Verfahren unter Verwendung derartiger Bindemoleküle vorgeschlagen, wobei sich die therapeutische Anwendung auf die Hemmung des Wachstums oder das Abtöten von Zellen bezieht, die solche membranständigen Immunglobuline des modifizierten Typs exprimieren.

Im Allgemeinen stellen Leukämien und Lymphome attraktive Ziele für die Behandlung mit Immuntoxinen und/oder Immuncytokinen dar. Die Reaktion von Patienten mit B-Zell-Malignitäten ist in klinischen Studien der Phase I/II der Immuntoxin-Aktivität umfassend untersucht worden (Amlot et al., (1993), Blood 82, 2624-2633; Sausville et al., (1995), Blood 85, 3457-3465; Grossbard et al., (1993), Blood 81, 2263-2271; Grossbard et al., (1993) Clin. Oncol. 11, 726-737). Bisher sind einige Antitumor-Reaktionen festgestellt worden, doch verhinderte eine Immuntoxin-vermittelte Toxizität gegenüber normalem Gewebe oftmals Dosis-Eskalationen auf therapeutische Mengen. Mehrere B-Zell-spezifische Antigene, wie etwa CD19, CD22 und CD40, wurden als Ziele von Immuntoxinen ausgewählt, die mit Pflanzentoxinen, wie etwa der Ricin A-Kette, und bakteriellen Toxinen, wie etwa Pseudomonas Exotoxin A (PE), erzeugt wurden (Uckun et al., (1992), Blood 79, 2201-2214; Ghetie et al., (1991), Cancer Res. 51, 5876-5880; Francisco et al., (1995), Cancer Res. 55, 3099-3104).

Für eine zielgerichtete, d.h. spezifische Immuntherapie stellen membranständige Immunglobuline gut geeignete Ziele dar. Während der B-Zell Entwicklung im Knochenmark generiert jeder einzelne B-Zell Vorläufer durch die Umlagerung von einzelnen GenSegmenten seinen eigenen und nahezu einzigartigen B-Zell Rezeptor (BCR).

Es sind zwei Varianten (Subset 2; Subset 4) des autonom aktiven BCRs bekannt, die sich hinsichtlich ihres jeweils charakterisierenden molekularen Motivs (Epitops) voneinander unterscheiden (Minici, C. et al., Distinct homotypic B-cell receptor interactions shape the outcome of chronic lymphocytic leukaemia, Nature Comm. (2017)). Beide Varianten weisen unterschiedliche kurze Aminosäuresequenzen auf, die für diese Varianten jeweils spezifisch sind. Dem Fachmann ist bekannt, dass neben den aufgeführten Subsets auch andere CLL-B-Zell Rezeptoren autonom aktiv sind. Der für die autonom aktive Funktionalität des Rezeptors maßgebliche Bereich von Subset 2 kennzeichnet sich hierbei durch die Aminosäuresequenzen KLTVLRQPKA (SEQ ID NO. 1) und VAPGKTAR (SEQ ID NO. 2) der leichten Kette, während der für die autonom aktive Funktionalität des Rezeptors maßgebliche Bereich von Subset 4 durch die Aminosäuresequenzen PTIRRYYYYG (SEQ ID NO. 3) und NHKPSNTKV (SEQ ID NO. 4) des variablen Teils der schweren Kette definiert wird. Die zur Erzeugung der murinen Antikörper im Rahmen der Immunisierung eingesetzten Sequenzen für die Subsets 2 und 4 sind in den SEQ ID NOS. 5 und 6 (vHC; LC) bzw. 7 und 8 (vHC; LC) angegeben. Der Vollständigkeit halber wird in SEQ ID NO. 17 (VSSASTKG) eine weitere Zielsequenz bzw. ein weiteres Epitop mit Spezifität für den variablen Teil der schweren Kette eines BCR des Subsets 4 angegeben. Neben den für die Ausbildung des autonom aktiven Zustand des BCR (Subset 4) verantwortlichen Zielsequenzen (Epitopen) gemäß SEQ ID NOS. 3 und 4 stellt die Sequenz gemäß SEQ ID NO. 17 somit eine weitere für diesen Subset charakteristische Eigenschaft dar.

Es wird darauf hingewiesen, dass das Auffinden und Charakterisieren der Subsets 2 und 4 als zwei Varianten des B-Zellrezeptors in Patienten mit kritischem Krankheitsverlauf auf der Untersuchung zahlreicher Einzelfallstudien beruht und daher nicht bedeutet, dass in einer möglichen Vielzahl anderer Subtypen des BCRs nicht dieselben vorliegend für die beiden bekannten Subtypen kennzeichnenden Zielsequenzen (Epitope) vorhanden sind und mit einem schweren Krankheitsverlauf korrelieren.

Obwohl sich Antikörper gegen beide dieser Subsets grundsätzlich mit Standardmethoden z.B. in Mäusen generieren lassen sollten, wurde überraschenderweise beobachtet, dass eine Immunisierung unter Verwendung von Peptiden nicht zur Bildung der gewünschten spezifischen Antikörper führt. Auch die Immunisierung unter Verwendung einzelner Ketten des Rezeptors, wie z.B. der Verwendung der die modifizierten Sequenzbereiche umfassenden leichten Kette des BCR, brachte nicht den gewünschten Erfolg, weshalb Mäuse schließlich mit der rekombinant hergestellten löslichen Form des BCRs (vgl. SEQ ID NOS. 5 und 6) immunisiert wurden. Aus diesen Mäusen konnten anschließend Immunzellen mit der gewünschten Spezifität gewonnen und durch Zellfusion in Hybridoma-Zellen transformiert werden. Hierbei zeigte sich überraschenderweise, dass die aktiven Antikörper nicht mittels ELISA Test oder anderen Standard-Verfahren identifiziert werden konnten. Die in einem ersten Schritt mittels ELISA als potenzielle Bindungspartner identifizierten Klone erwiesen sich jedoch nach der Selektion als entweder unspezifisch bindend oder nicht an den autonom aktiven Rezeptor (einschließlich der SEQ ID NOS. 1 und 2) bindend und mussten daher verworfen werden.

Die bis zu dieser Erkenntnis eingesetzten Verfahren umfassten nicht nur Standardmethoden wie ELISA und SPR, sondern auch die intrazelluläre Expression in Fibroblasten mit einer intrazellulären FACS-Färbung als Bindekontrolle.

Nach aufwendigen weiteren Versuchsreihen konnte gezeigt werden, dass eine erfolgreiche Selektion erfindungsgemäß geeigneter Bindemoleküle weder mit freien Rezeptoren oder deren Fragmenten noch mit membranständigen oder intrazellulären Rezeptorfragmenten durchgeführt werden kann. Stattdessen wurde beobachtet, dass die Selektion nur unter Verwendung eines Zellsystems gelang, im Rahmen dessen der komplette und funktionelle B-Zell Rezeptor membranständig präsentiert wurde. Dabei ist es von großer Bedeutung, dass der BCR mit seinen modifizierten Bereichen (Epitope) in bzw. auf diesen Zellen autonom aktiv vorliegt bzw. präsentiert wird. Nur mit dieser Vorgehensweise, deren Bedingungen ein weitgehend physiologischnatives *in situ* Szenario widerspiegeln, konnte ein Antikörper identifiziert werden, der hochspezifisch und selektiv lediglich an die Tumorzellen bindet, d.h. an B-Zellen, die einen BCR mit einem Epitop auf ihrer Zellmembran exprimieren, welches für den Subset-2 oder Subset-4 dieses Zelltyps charakteristisch ist, nicht jedoch an andere B-Zellen oder deren Rezeptoren (BCRs), die definitionsgemäß keine B-Zellen des Subsets 2 oder 4 darstellen. Mit anderen Worten bindet das erfindungsgemäße Bindemolekül selektiv an autonom aktive oder autonom aktivierte B-Zell Rezeptoren, die durch das Vorhandensein von strukturellen Domänen oder Epitopen (Zielsequenzen) charakterisiert und für den autonom aktiven oder aktivierten Zustand der B-Zell Rezeptoren ursächlich sind. Das selektive Bindungsverhalten des erfindungsgemäßen Bindemoleküls bedeutet, dass es nicht an Rezeptoren oder andere Membranstrukturen von B-Zellen bindet, die keine strukturelle Domäne oder kein Epitop aufweisen, die für den autonom aktiven oder aktivierten Zustand der B-Zellen ursächlich sind. Somit bindet das erfindungsgemäße Bindemolekül nicht selektiv an Zielsequenzen des B-Zell Rezeptors, die für den Subset 2 oder den Subset 4 nicht charakteristisch sind, und insbesondere nicht an einen B-Zell Rezeptor, der keine der Sequenzen SEQ ID NOS. 1, 2, 3 und/oder 4 aufweist.

Es konnte ferner gezeigt werden, dass die Verwendung von arretierten pro-/prä-B-Zellen, die aus 'Triple Knockout'-Mäusen (TKO) gewonnen wurden, trotz der schwierigen Handhabung und der aufwendigen Gewinnung besonders gut geeignet sind, diese Rezeptoren zu exprimieren und im Rahmen eines Testsystems zur Identifikation dieser Rezeptoren verwendet zu werden. Das Stadium der pro-/prä-B-Zellen ist natürlicherweise dazu ausgelegt, die Reifung und Selektion der BCRs durchzuführen, und die Zellen dieses Stadiums sind aufgrund ihrer Enzymausstattung (Chaperone etc.) besonders geeignet, auch "schwierige" BCR-Komponenten korrekt zu falten und auf Ihrer Oberfläche in hinreichend physiologisch nativer Form darzustellen. Durch die nachfolgend dargelegten Deletionen (Knockouts) wird verhindert, dass durch eine Rekombination oder die Verwendung der Surrogaten Leichten Kette ("surrogate light chain") eine Veränderung des gewünschten BCR stattfindet. Durch Verwendung dieser Zellen oder dieses Zelltyps arretierter pro-/prä-B-Zellen zur Expression und Präsentation von BCRs im Rahmen einer Selektion von Antikörpern mit selektiv-spezifischem Bindungsverhalten gegenüber autonom aktiven oder aktivierten B-Zell Rezeptoren wird eine Selektionsplattform bereitgestellt, die im Vergleich mit den im Stand der Technik herkömmlich zur Selektion eingesetzten Systemen durch eine sehr viel höhere Qualität gekennzeichnet ist, welche den hohen Aufwand der Verwendung von primären TKO Zellen, bzw. deren Kultivierung über wenige Passagen rechtfertigt.

Nach der zuvor beschriebenen Selektion geeigneter Hybridomazellen konnten die für prophylaktische und/oder therapeutische Zwecke geeigneten Antikörper in Form monoklonaler Antikörper in größeren Mengen gewonnen werden. Durch Sequenzierung der DNA dieser Zellen konnte die Bindungsstelle des Antikörpers ermittelt werden (vgl. SEQ ID NOS. 9 und 10). Entsprechende Verfahren sind dem Fachmann bekannt und sind auch kommerziell verfügbar. Hierbei ist es vorteilhaft, wenn man eine größere Zahl an Hybridomazellen gewinnt und diejenigen mit der besten Bindeaktivität (Spezifität und Bindestärke/Affinität) auswählt.

Durch die somit erhaltene genetische Information über die Bindungsstelle wurde die dafür kodierende Sequenz in ein Expressionsplasmid mit der DNA einer humanen Antikörpersequenz eingefügt, um auf üblichem Weg der Rekombination einen humanisierten monoklonalen Antikörper mit der gewünschten Spezifität zu erzeugen. Diese humanisierten Antikörper zeigten aufgrund ihrer einzigartigen Spezifität eine im Vergleich mit herkömmlichen Wirkstoffen bessere prophylaktische und therapeutische Wirksamkeit bei vergleichsweise sehr geringen Nebenwirkungen. Dem Fachmann ist klar, dass sich diese humanisierten Antikörper auf biotechnologischem Weg in großen Mengen herstellen lassen. Zur Aufreinigung der synthetisierten Antikörper können standardisierte Verfahren angewendet werden, wie z.B. Kombinationen aus Präzipitation, Filtration und Chromatographie, was dem Fachmann hinreichend bekannt ist, wobei beachtet werden sollte, die Antikörper nicht zu denaturieren und mögliche Fremdsubstanzen wie z.B. Proteine, Pyrogene und Toxine quantitativ zu entfernen.

Bevorzugt werden die gewünschten Antikörper in Systemen exprimiert, in denen der Antikörper eine Glykosylierung wie insbesondere eine humane Glykosylierung erfährt. Solche Systeme sind dem Fachmann hinreichend bekannt und schließen die Verwendung von Insektenzellen (S2-Zellen), Säugerzellen (CHO-Zellen) und, besonders bevorzugt, humane Zellen wie zum Beispiel Zellen des Typs HEK293T ein.

Der hinreichend aufgereinigte Antikörper kann an sich schon therapeutisch wirksam sein, sofern er einen Isotyp aufweist, der eine spezifische Immunantwort hervorruft, wie z.B. ein IgG Subtyp, der über Fc-Rezeptoren zu einer Immunantwort gegen den Tumor führt.

Der Antikörper kann aber auch als Fragment vorliegen. Dabei ist es wichtig, dass die Antigen-Bindestelle in dem Fragment vorliegt, es sich also um ein funktionales Fragment handelt. Solche Fragmente lassen sich z.B. durch Protease-Behandlung als F(ab) Fragmente herstellen. Da diese Fragmente im konstanten Teil des Antikörpers trunkiert sind, ist es hier vorteilhaft und deshalb bevorzugt, ein Effektormolekül zum Abtöten von Neoplasien einzufügen.

Nach einer alternativ bevorzugten Ausführungsform ist der Antikörper mit einem Konjugat versehen, um dessen Wirkung zu steigern. Dieses Konjugat ist ein Bereich zum Abtöten von Neoplasien und kann solche Neoplasien entweder als direkte oder indirekte Wirkung abtöten. Ein Beispiel für ein solches Konjugat ist die Anbindung von Ricin an den Antikörper, wobei dessen bevorzugt kovalente Anbindung z.B. durch Verwendung chemischer Crosslinker durchgeführt wird. Derartige Moleküle und Verfahren sind in dem Buch Bioconjugate Techniques von Greg T. Hermanson im Kapitel 11 Immunotoxin Conjugation Techniques umfänglich beschrieben.

Nach einer weiteren bevorzugten Ausführungsform kann der Antikörper auch in modifizierter Form wie z.B. als biologisches Bindemolekül in Form eines Fusionsproteins mit T-Zell spezifischen Aktivierungsdomänen vorliegen. Zur Generierung dieses sogenannten chimären Antigen-Rezeptors (Chimeric Antigen Receptor; CAR) werden zunächst T-Zellen aus dem peripheren Blut des Patienten gewonnen und *in vitro* derart gentechnisch verändert, dass sie den CAR auf ihrer Zelloberfläche exprimieren. Anschließend werden diese modifizierten T-Zellen wieder in den Patienten eingebracht, wodurch somit eine CAR-T-Zell Immuntherapie durchführbar ist (siehe z.B. N Engl J Med. 2014 Oct 16; 371(16):1507-17. doi:10.1056/NEJMoa1407222).

Zur therapeutischen Verwendung mittels Verabreichung (Infusion) wird der Antikörper bevorzugt in einer Zusammensetzung eingesetzt, die einen pharmazeutisch verträglichen Träger aufweist.

Ein pharmazeutisch verträglicher Träger ist ein Träger, der für einen behandelten Patienten physiologisch vertraglich ist und die therapeutischen Eigenschaften der Verbindung, mit welcher er verabreicht wird, bewahrt. Ein beispielhafter pharmazeutisch verträglicher Träger ist physiologische Kochsalzlösung. Andere geeignete physiologisch verträgliche Träger und deren Formulierungen sind dem Fachmann bekannt und werden beispielsweise beschrieben in Remington's Pharmaceutical Sciences (18. Ausgabe), Hrsg. A. Gennaro, 1990, Mack Publishing Company, Easton, PA).

Zur stabilen. Lagerung kann es von Vorteil sein, den Antikörper oder dessen Fragmente in einer stabilisierten Form bereitzustellen, was dementsprechend auch bevorzugt ist. Dazu kann z.B. eine Trocknung mit einem stabilisierenden Salzpuffer erfolgen. Ein solcher Puffer kann z.B. eine Phosphat-gepufferte Salzlösung (PBS) sein, wie dem Fachmann bekannt ist. Eine geeignete Trocknungsform ist z.B. Gefriertrocknen oder Lyophilisieren.

Eine weitere therapeutische Anwendungsmöglichkeit der erfindungsgemäßen Bindemoleküle stellt das an sich bekannte Verfahren der Apharese dar, bei der eine Behandlung des Blutes oder einer Blutprobe eines Patienten außerhalb seines Körpers im Sinne einer "Blutwäsche" erfolgt.

Beispielsweise kann der erfindungsgemäße Antikörper mit Spezifität für den autonom aktivierten BCR (Subset-2) in einem Apharesesystem zur Abtrennung von Leukämiezellen aus einer Blutprobe eines Patienten verwendet werden. Hierzu sind grundsätzlich verschiedene Verfahren geeignet, wie dem Fachmann bekannt ist.

Nach einer ersten beispielhaften Ausführungsform können die Antikörper an magnetisierbare Partikel (Beads) (z.B. Dynabeads) gebunden sein. Das Blut wird mit einem Antikoagulans versehen und außerhalb des Körpers des Patienten mit den Partikeln in Kontakt gebracht. Idealerweise wird dazu pro Tumorzelle mindestens ein Partikel, vorzugsweise 10 bis 100 Partikel eingesetzt. Ein Partikel mit einer Größe von beispielsweise kleiner 20 µm enthält dabei typischerweise mehrere Antikörper derselben Spezifität (Anzahl der Partikel größer 5000/µl Blut). Mit Hilfe von Magneten können diese Partikel dann gebunden werden, bevor das gereinigte, restliche Blut dem Patienten rückgeführt wird. Durch diese therapeutische Maßnahme wird die Anzahl der Tumorzellen im Blut des Patienten deutlich reduziert. Nach einer anderen Ausführungsform haben die Partikel Größen von mehr als 20 µm und weisen ebenfalls eine Vielzahl von Antikörpern pro Partikel auf (> 100, > 1000). So können mit einem Partikel viele Lymphozyten (Tumorzellen) gebunden und entfernt werden. Diese Partikel/Zell-Konjugate werden durch klassische Zentrifugation, wie sie bei der Apharese üblicherweise verwendet wird, entfernt. Die dafür notwendigen Zeiten hängen von der Art der Partikel und der Apparatur ab und müssen experimentell ermittelt werden.

Im Rahmen einer weiteren Ausführungsform können sowohl die Partikel/Zell-Konjugate (insbesondere bei Verwendung großer Partikel über 20 µm Durchmesser) als auch freie Partikel ohne Zellbindung mit Hilfe eines feinen Netzwerks von Blut abgetrennt werden. Solche Netzwerke sind z.B. als sog. 'Cellstrainer' kommerziell erhältlich. Verfahren zum Konjugieren der Antikörper an die Partikel sind dem Fachmann hinreichend bekannt. Verfahrensanweisungen werden z.B. von der Firma Dynal ihren Kunden zur Verfügung gestellt.

Einzelne Aspekte der vorliegenden Erfindung werden nachfolgend anhand von Beispielen naher erläutert.

Bevor detaillierte Erläuterungen zum experimentellen Vorgehen dargelegt werden, sei auf die nachfolgenden Ausführungen verwiesen.

Die Herstellung und Identifizierung von Antikörpern, die selektiv an die modifizierten B-Zell Rezeptoren binden, war von größeren und unvorhergesehenen Problemen gekennzeichnet. Die Erzeugung der Hybridome erfolgte mittels Standardmethoden. Der Überstand von den Hybridomgruppen wurde gepoolt und mittels ELISA auf positive Bindungsereignisse untersucht (lösliche B-Zell Rezeptoren auf der ELISA-Platte). Positive Pools wurden vereinzelt und die individuellen Klone getestet. Dabei wurden im ELISA überraschenderweise keine positiven Klone mehr identifiziert. Die positiven ELISA Signale der Poole stellten sich im Nachhinein als unspezifische Bindungen heraus.

Um bessere Epitope für die Erkennung der Antikörper zu schaffen, wurde nun die leichte Kette des BCRs in Fibroblasten exprimiert. Dies sollte die korrekte Faltung des Proteins gewährleisten, welches das für das autonome Signal verantwortliche Motiv (Epitop) trägt. Mit diesen Zellen wurden intrazelluläre FACS-Analysen durchgeführt. Es konnte kein positiver Klon (Antikörper) identifiziert werden.

Aus diesem Grund wurden in einem weiteren Experiment RAMOS Zellen (humane Burkitt Lymphom Zellline) modifiziert, so dass diese funktionelle modifizierte BCRs aufwiesen. Damit sollte die vollständig korrekte Biosynthese, Faltung und Modifikation des BCRs gewährleistet sein. Dazu wurde der zelleigene BCR mittels CRISPR deletiert und dann der "CLL-Rezeptor" molekularbiologisch rekonstituiert (Elektroporation von CMV-Vektoren). Diese Zellen wurden zum Testen von positiven Bindungsereignissen verwendet. Auch hier war mittels FACS kein positiver Klon nachweisbar.

Überraschenderweise brachte dagegen die Verwendung von murinen TKO ('Triple Knock-out') Zellen (arretierte pro-/prä-B-Zellen), in die der CLL-Rezeptor mittels eines Genshuttles eingebracht wurde, einen positiven Klon. Und das, obwohl das humane Zellsystem dies nicht gewährleisten konnte. Diese Zellen weisen als Besonderheit folgende drei Knockouts in ihrem Genom auf:
- der Knockout von RAG2 verhindert die somatische Rekombination von eigenen schweren und leichten Immunglobulinketten, weshalb die endogene Bildung eines BCR ausgeschlossen ist. Dies führt zu einer Arretierung, einem Blockieren oder 'Einfrieren' entsprechend behandelter B-Zellen in diesem Entwicklungsstadium. Es ist bekannt, dass RAG1 und RAG2 einen Komplex bilden, der das übliche VDJ-Rearrangement erst ermöglicht, weshalb ein Knockout von RAG1 ein gleich wirkendes Mittel und somit eine Alternative zum Knockout von RAG2 darstellt und von der erfindungsgemäßen Lehre umfasst ist.
- die Deletion von Lambda5, einem Teil der Surrogaten Leichten Kette, verhindert die Formation eines prä-BCR. Da der prä-BCR autonom aktiv ist, würde dies den Nachweis eines autonom aktiven Rezeptors stören. Da hier ein neuer BCR in die Zelle kloniert wird, ist ein prä-BCR unerwünscht, da dieser mit der erwünschten Schweren Kette (HC) in Verbindung mit der unerwünschten Surrogaten Leichten Kette auf der Oberfläche erscheinen und die Selektion stören würde.
- der Knockout von SLP65, einem Adapterprotein von zentraler Bedeutung im BCR-Signalweg, verhindert die Aktivierung der TKO Zelle durch einen eventuell rekonstruierten BCR.

Die Kombination der Knockouts von RAG2 oder RAG1 und Lambda5 führt zu einer Blockade im Übergang von dem pro-B Zell-Stadium hin zum prä-B Zell-Stadium, welches klassischerweise mit der beginnenden Umlagerung der VDJ-Segmente der schweren Kette (HC) gekennzeichnet ist. Daher handelt es sich um pro-/prä-B Zellen.

Für die Expression des BCR und Selektion des geeigneten Antikörpers ist der Knockout von RAG2 oder RAG1 und Lambda5 ausreichend. Durch die Rekonstitution mit dem induzierbaren SLP65 kann die Aktivität des BCR gemessen werden.

Die Methode der Wahl hierbei ist die Messung des Ca-flux nach Induktion des SLP65 mittels FACS-Analyse und der Verwendung eines Ca²⁺ abhängigen Farbstoffes wie Indo-1. Diese Methoden sind dem Fachmann bekannt (s. (M. Dühren-von Minden et. al; Nature 2012).

Durch die ersten beiden Knockouts wurde sichergestellt, dass nur der "BCR of Interest" auf der Oberfläche exprimiert wird. Durch die Verwendung eines induzierbaren SLP65, mit welchem die Zellen rekonstituiert wurden, können überdies die Funktion der exprimierten BCRs charakterisiert und somit der autonom aktive Zustand der BCRs auf der Oberfläche vor der Selektion verifiziert werden.

Die Expression des BCRs wurde mittels anti-IgM und anti-LC Antikörper am FACS bestimmt. Dazu wurden einige Zellen abgenommen und mit je 5µl Antikörper in einem Gesamtvolumen von 100µl in PBS gefärbt.

Mit diesen Zellen als "Target" gelang es nun mittels FACS, einen Antikörper zu identifizieren, der an den modifizierten Bereich, durch welchen die autonome Aktivierung des BCR begründet und gekennzeichnet ist, spezifisch bindet. Und dies, obwohl eine Bindung an den gleichen Rezeptortyp in RAMOS Zellen nicht erfolgreich war!

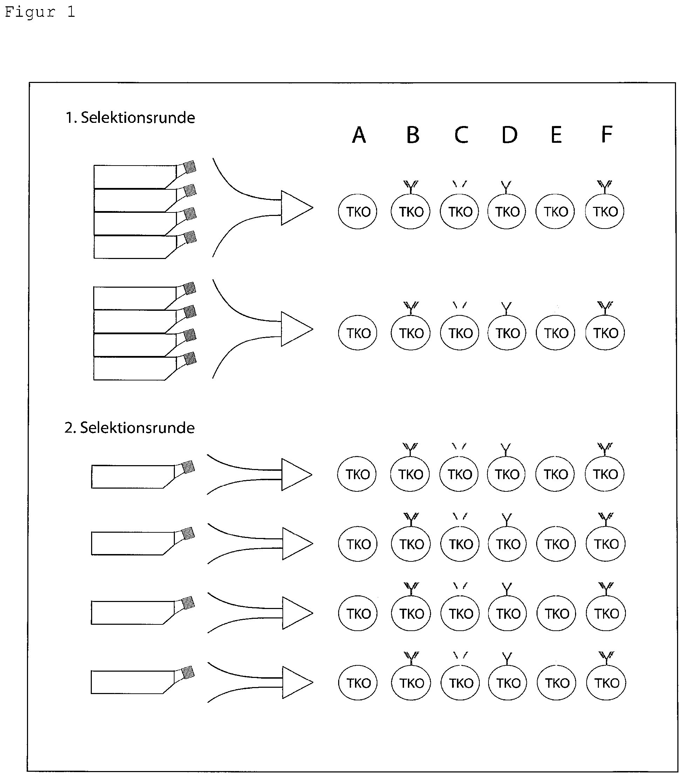

Dazu wurden die Zellen, welche den "BCR of Interest" auf der Oberfläche trugen, zunächst mit den gepoolten Überständen inkubiert, und nach einigen Waschschritten wurden die gebundenen Antikörper mittels Sekundärantikörpern detektiert. Für eine spezifische Selektion wurden TKO-Zellen (TKOs) verwendet, welche verschiedene Versionen des "BCR of Interest" exprimierten. Die in Figur 1 dargestellte Selektionsmatrix ist exemplarisch für die Selektion eines CLL-Subset 2 BCRs und diente der Identifizierung und Selektion positiver Klone. Zur einfacheren Identifikation wurden die Überstände der Hybridome gepoolt und gemessen. Die Gruppen, die eine Bindung zeigten, wurden vereinzelt, und die Überstände der jeweiligen Hybridome auf Bindung getestet.

Die Bestätigung, dass der selektierte Antikörper spezifisch an den modifizierten BCR und nicht an andere BCR Varianten bindet, erfolgte mittels zweier Blindproben, d.h. mit Zellen ohne BCR (s. Figur 1A) sowie mit Zellen mit Non-CLL-BCR (s. Figur 1E). Primäre B-Zellen aus dem Blut von Leukämiepatienten wurden mittels FACS auf Bindung untersucht. Der selektionierte Antikörper war in der Lage, spezifisch diejenigen BCRs zu identifizieren, welche die Zielstruktur aufwiesen. Dies wurde auf genomischer Ebene bestätigt. Proben ohne diese Zielstruktur wiesen keine Bindung auf.

Die Erfindung wird nachfolgend anhand von Beispielen unter Berücksichtigung der Figur 1 näher erläutert.

### Beispiel 1

Den Ausgangspunkt für die Herstellung von Triple-Knockout-Zellen (TKO) bilden transgene Mäuse, welche einen jeweiligen Knockout für die Gene Lambda5, RAG2 und SLP65 aufweisen (Dühren von Minden et al., 2012, Nature 489, p309-313). Die Erstellung solcher Mäuse ist dem Fachmann bekannt und gehört zum Stand der Technik. Für die Gewinnung der Zellen wurde den Mäusen nach ihrer Tötung das Knochenmark der Oberschenkelknochen extrahiert. Die so gewonnenen Zellen wurden anschließend unter Bedingungen in Kultur genommen, die das Überleben von pro-/prä-B-Zellen begünstigen (37°C, 7,5% CO2, Iscoves Medium, 10% FCS, P/S, murinem IL7). Nach mehreren Passagen wurden zur Kontrolle eine FACS-Sortierung durchgeführt, die pro-/prä-B-Zellen sortiert und anschließend wieder in Kultur genommen. Die dazu verwendeten Marker sind dem Fachmann bekannt.

Für die Rekonstitution mit einem 'BCR of Interest' wurden die entsprechenden, für die schweren (HC) und leichten (LC) Ketten kodierenden Sequenzen synthetisiert und dann jeweils in einen CMV-Promotor aufweisende Expressionsvektoren kloniert. Diese wurden mittels Lipofektion in die Verpackungszelllinie (Phoenix-Zelllinie) eingebracht. Nach einer 36 Stunden andauernden Inkubation wurde der Virusüberstand abgenommen und für eine Spinfektion der TKO Zellen verwendet. Sowohl die Arbeiten zur Gewinnung der Überstände als auch die Spinfektion der TKO sind weitläufig bekannte Verfahren und dem Fachmann bekannt.

Die strukturellen Besonderheiten von Subset-2 B-Zell Rezeptoren wurden der entsprechenden Literatur entnommen (s.o.). Exemplarische CLL-Subset 2 VH und vollständige LC DNA-Segmente wurden von einem Lohnhersteller in einem Standardverfahren synthetisiert. Diese wurden dann mit einem murinen IgG1-Konstantensegment mittels PCR fusioniert und in einen CMV-Vektor kloniert. Die Sequenz des fertigen Vektors wurde mittels Sanger-Sequenzierung bestätigt.
CLL-Subset 2 VH (SEQ ID NO. 5): 
EVQLVESGGGLVKPGGSLRLSCAASGFTFRSYSMNWVRQAPGKGLEWVSSIISSSSYIYYADS
VKGRFTISRDNAKNSLYLQMNSLRAEDTALYYCARDQNAMDVWGQGTTVTVSS
CLL-Subset 2 LC (SEQ ID NO. 6):

SYELTQPPSVSVAPGKTARITCAGNNIGSKSVHWYQQKPGQAPVLVIYYDSDRPSGIPERFSG
SNSGNTATLTISRVEAGDEADYYCQVWDSGSDHPWVFGGGTKLTVLRQPKAAPSVTLFPPSSE
ELQANKATLVCLISDFYPGAVTVAWKADSSPVKAGVETTTPSKQSNNKYAASSYLSLTPEQWK
SHRSYSCQVTHEGSTVEKTVAPTECS

Für die Expression des CLL-Subsets 2 IgG1 wurde ein humanes zelluläres Expressionssystem basierend auf HEK293T Zellen genutzt. Für die Transfektion wurde ein auf Polyethylenimin (PEI) basierendes Protokoll angewendet. Nach mehreren Passagen wurde der Überstand gepoolt, und das in dem vereinigten Zellüberstand enthaltende Medium wurde mittels Protein G Säulchen aufgereinigt. Die Reinheit und Qualität der löslichen Subset-2 IgG1 wurde mittels Westernblot bestimmt.

Die Herstellung von monoklonalen Antikörpern erfolgte nach dem Standardverfahren in Mäusen und der anschließenden Generierung von Hybridoma-Zellen. Das Screening nach positiven Klonen erfolgte nicht wie herkömmlich mittels ELISA. Da es sich bei der Zielstruktur um einen membranständigen Rezeptor handelt, ist es von zentraler Bedeutung, die Bindung der potentiellen Antikörper auch in einem zellulären System, d.h. unter weitgehender Wahrung der für diesen Zelltyp nativen zellphysiologischen Zustande, zu validieren. Es wurden zunächst Gruppen von gepoolten Überständen mittels FACS-Analyse auf Bindungsereignisse untersucht. Dazu wurden verschiedene CLL-Subset 2 BCR Varianten auf der Oberfläche einer Zelllinie (TKO) exprimiert, welche selber keinen BCR exprimieren kann. So konnten zunächst die Überstände identifiziert werden, deren Antikörper eine Bindung aufzeigten. Anschließend wurden die Überstände der einzelnen Hybridom-Klone hinsichtlich ihrer Bindung eingehender untersucht, um auf diese Weise hochspezifische Klone mit hoher Affinität zu identifizieren.

Für das Screening-Verfahren wurden im Rahmen der vorhergehenden Transformation unterschiedliche Vektoren für die folgenden Kombinationen aus schwerer Kette (HC) und leichter Kette (LC) der entsprechenden CLL-BCRs eingesetzt, wobei diese Kombinationen auf der Oberfläche des BCR-Rekonstitutionssystems verwendet wurden:
- Kontrolle (Transformationsvektor ohne BCR)(s. Fig.1A)
- Vektor mit für den CLL-Subset 2 typischer HC / LC (s. Fig. 1B)
- Vektor mit einer non-CLL-Subset 2 HC / einer für den CLL-Subset 2 typischen LC (ohne Zielmotiv; Epitop) (s. Fig. 1C)
- Vektor mit für den CLL-Subset 2 typischer HC / einer non-CLL-Subset 2 LC (s. Fig. 1D)
- Vektor mit einer non-CLL-Subset 2 HC / einer non-CLL-Subset 2 LC (s. Fig. 1E)
- Vektor mit für den CLL-Subset 2 typischer HC / LC (einschließlich Mutation R110G (Zielmotiv)) (s. Fig. 1F).

Diese Vorgehensweise der Selektion ist in Figur 1 schematisch am Beispiel der CLL-Subset 2 BCRs veranschaulicht, wobei sich die Bezeichnung 'TKO' auf TKO-Zellen (s.o.) bezieht.

In der 1. Selektionsrunde wurden Überstände mehrerer Klone vereinigt und hinsichtlich ihres Bindungsprofils an die Selektionsmatrix untersucht. Ein positives Bindungsprofil ist gegeben, wenn sich eine spezifische Bindung an den "BCR-of-Interesst" zeigt. Gruppen, die ein solches Profil zeigten, wurden vereinzelt, und das Bindungsprofil der einzelnen Klone wurde im Rahmen einer zweiten Selektionsrunde nochmals auf der Selektionsmatrix charakterisiert. Die Bindung der monoklonalen Antikörper wurde unter Anwendung eines FACS-Bindungsassays unter Verwendung eines Fluoreszenz-markierten Anti-Mouse-IgG Antikörpers verifiziert. Es bezeichnen: A) kein BCR (Kontrolle); B) ein CLL-Subset2 typischer BCR; C) ein BCR mit willkürlicher schwerer Kette und einer CLL-Subset2 typischen leichten Kette; D) ein BCR mit einer CLL-Subset2 typischen schweren Kette und einer willkürlichen leichten Kette; E) ein BCR mit willkürlicher schwerer und leichter Kette (Kontrolle; nicht CLL-Subset2 typischer BCR); F) ein CLL-Subset2 typischer BCR mit einer Mutation im Zielmotiv (R110G)(Kontrolle).

Aufgrund des Befundes, dass der Antikörper nur an die Zellen mit den Zielstrukturen bindet (CLL-Subset2 BCR; Fig. 1B), kann gefolgert werden, dass hier ein Antikörper vorliegt, der spezifisch an Zellen mit autonom aktiven Rezeptoren bindet.

Hierbei zeigte sich, dass die Verwendung von Zellen, die sich im pro-/prä-Stadium der B-Zell Entwicklung befinden, für die zum Nachweis benötigte exakte Expression des BCR notwendig ist. Diese Zellen sind entwicklungsgenetisch eingerichtet, um neue BCR durch exakte Faltung und Expression auf ihrer Oberfläche darzustellen. Durch die Inaktivierung (Knockout) von RAG2 und Lambda5 wird die Expression eines endogenen BCR bzw. prä-BCR verhindert. Die Deletion von SLP65 und die anschließende Rekonstruktion eines induzierbaren SLP65 ermöglicht es, das Aktivitätsniveau des "BCR of Interest" zu charakterisieren.

Zur Bestimmung der Aminosäurensequenz der mittels Selektion ausgewählten monoklonalen Antikörper wurde aus den einzelnen Hybridom-Klonen die mRNA isoliert, daraus cDNA generiert und diese mittels Anchor-PCR amplifiziert (Rapid expression cloning of human immunoglobulin Fab fragments for the analysis of antigen specificity of B cell lymphomas and anti-idiotype lymphoma vaccination; Osterroth F, Alkan O, Mackensen A, Lindemann A, Fisch P, Skerra A, Veelken H., J Immunol Methods 1999 Oct 29; 229(1-2):141-53).

Nach Identifizierung und Sequenzbestimmung der für die Bindung wichtigen Bereiche (CDRs) wurden diese mittels PCR auf humanes Antikörpergerüst übertragen. Dazu wurde die VH-Sequenz aus den humanen FR-Regionen und den murinen CDR Regionen *in silico* generiert und anschließend als DNA-Fragmente synthetisiert. Diese wurden anschließend mittels PCR mit einem humanen IgG1 fusioniert und in einen für die Expression geeigneten Vektor kloniert.

Für die Generierung der monoklonalen Antikörper wurden neben den kompletten Immunglobulinen auch synthetische Peptide verwendet, welche die Regionen für die Fähigkeit eines autonomen Signals repräsentierten.

Der spezifische monoklonale Antikörper gegen Subset-2 wurde sequenziert. Dabei wurden die folgenden Aminosäurequenzen bestimmt, wobei die SEQ ID NO. 9 den variablen Teil der schweren Kette (HC), und die SEQ ID NO. 10 den variablen Teil der leichten Kette (LC) betreffen, und wobei die markierten Bereiche - in der angegebenen Reihenfolge - CDR 1, 2 und 3 bezeichnen.
SEQ ID NO. 9 (AVA-mAb01 HC) 
QVQLQQSGPGLVQPSQSLSITCTVSGFSLTSYGIHWVRQSPGKGLEWLGVIWRGGGTDSNAAF
MSRLSITKDNSKSQVFFKMNSLQADDTAIYYCARSRYDEEESMNYWGQGTSVTVSS
SEQ ID NO. 10 (AVA-mAb01 LC)

QIVLTQSPASLSASVGETVTITCRASGNIHSYLAWYQQKQGKSPQLLVYNAKTLADGVPSRFS
GSGSGTQYSLKINSLQPEDFGSYYCQHFWNTPPTFGAGTKLELK

Die mit CDR1, CDR2 und CDR3 korrespondierenden Teilsequenzen der schweren Kette gemäß SEQ ID NO. 9 sind in den SEQ ID NOS. 11 bis 13 angegeben, während die mit CDR1, CDR2 und CDR3 korrespondierenden Teilsequenzen der leichten Kette gemäß SEQ ID NO. 10 in den SEQ ID NOS. 14 bis 16 dargestellt sind.
SEQ ID NO. 11 (AVA-mAB01 CDR1 HC)
   GFSLTSYG
SEQ ID NO. 12 (AVA mAB01 CDR2 HC)
   IWRGGGT
SEQ ID NO. 13 (AVA mAB01 CDR3 HC)
   ARSRYDEEESMNY
SEQ ID NO. 14 (AVA mAB01 CDR1 LC)
   GNIHSY
SEQ ID NO. 15 (AVA mAB01 CDR2 LC)
   NAKT
SEQ ID NO. 16 (AVA mAB01 CDR3 LC)
   QHFWNTPPT

Die oben beschriebene Vorgehensweise ist exemplarisch für die Generierung von gegenüber CLL-Subset 2 spezifischen Antikörpern. Der gleiche Prozess wurde auch unter Verwendung von spezifischen Sequenzen und Isotypen für Subset 4 durchgeführt.

Exemplarische CLL-Subset 4 VH und vollständige LC DNA-Segmente wurden von einem Lohnhersteller in einem Standardverfahren synthetisiert. Diese wurden dann mit einem murinen IgG1-Konstantensegment mittels PCR fusioniert und in einen CMV-Vektor kloniert. Die Sequenz des fertigen Vektors wurde mittels Sanger-Sequenzierung bestätigt.
CLL-Subset 4 HC (SEQ ID NO. 7):

QVQLQQWGAGLLKPSETLSLTCAVYGGSFSGYYWTWIRQSPGKGLEWIGEINHSGSTTYNPSL
KSRVTISVDTSKNQFSLKLNSVTAADTAVYYCARGYGDTPTIRRYYYYGMDVWGQGTTVTVSS
ASTKGPSVFPLAPSSKSTSGGTAALGCLVKDYFPEPVTVSWNSGALTSGVHTFPACLQSSGLY
SLSSVVTVPSSSLGTQTYICNVNHKPSNTKVDKKC

Die fett markierten Bereiche bezeichnen die Zielsequenzen (Epitope) des variablen Teils der schweren Kette des BCR des Subsets 4, die für dessen autonom aktiven Zustand verantwortlich sind (vgl. SEQ ID NOS. 3 und 4).
CLL-Subset 4 LC (SEQ ID NO. 8):

DIVMTQSPLSLPVTLGQPASISCRSSQSLVHSDGNTYLNWFQQRPGQSPRRLIYKVSDRDSGV
PDRFSGSGSGTDFTLKISRVEAEDVGLYYCMQGTHWPPYTFGQGTKVEIKRTVAAPSVFIFPP
SDEQLKSGTASVVCLLNNFYPREAKVQWKVDNALQSGNSQESVTEQDSKDSTYSLSSTLTLSK
ADYEKHKVYACEVTHQGLSSPVTKSFNRGEC.

### Beispiel 2

Der erfindungsgemäße Antikörper mit Spezifität für den autonom aktivierten BCR (Subset-2) wurde in einem Apharesesystem zur Abtrennung von Leukämiezellen aus einer Blutprobe eines Patienten verwendet.

Einem Patienten wurde peripheres Blut entnommen (EDTA Blut aus Blutentnahmeröhrchen). Die Bestimmung die Lymphozytenzahl erfolgte mit einem Zellcounter und ergab 80.000 Lymphozyten/µl. Zur Bestimmung des "Tumorload", also der Belastung der Probe mit Tumorzellmaterial, wurde eine Immunphänotypisierung per FACS durchgeführt (mit dem CLL-Standard-Panel WHO- Tumorload). Sekundär wurden diejenigen Zellen, die das CLL-Epitop tragen, mit dem erfindungsgemäßen Antikörper angefärbt, um nachzuweisen, dass die Zellen für dieses Epitop positiv sind. Anschließend wurden 500 µl dieses Blutes mit 5 x 108 MACS MicroBeads (Miltenyi Biotech), die mit dem spezifischen Antikörper konjugiert waren, gemischt. Die Mischung wurde 5 Minuten lang bei Raumtemperatur geschüttelt, bevor das Blut über Miltenyi LS Säulen aufgearbeitet wurde. Hierbei verblieben die mit den Partikeln (Beads) konjugierten Lymphozyten auf der Säule, sodass aus der Säule als Durchfluss weitgehend von Lymphozyten befreites Blut gewonnen wurde. Nach zweifacher Aufreinigung über die Säulen (nach Vorschrift des Herstellers) konnte in einer FACS Messung eine Lymphozytenzahl von unter 5000 Lymphozyten/µl festgestellt werden. In einem Kontrollexperiment unter Verwendung von Blut eines CLL-Patienten ohne das Vorhandensein der autonom aktiven Variante des BCR, erbrachte diese Aufreinigung wie erwartet keine signifikante Reduktion der B-Zellen im Blut.

## Patentansprüche

1. Biologisches Bindemolekül, welches selektiv an autonom aktive oder autonom aktivierte B-Zell Rezeptoren bindet, wobei die autonom aktiven oder aktivierten B-Zell Rezeptoren durch das Vorhandensein von strukturellen Domänen oder Epitopen charakterisiert sind, an die das Bindemolekül selektiv bindet und die für den autonom aktiven oder autonom aktivierten Zustand der B-Zell Rezeptoren ursächlich sind.

2. Biologisches Bindemolekül nach Anspruch 1, **dadurch gekennzeichnet, dass** es selektiv an Zielsequenzen des B-Zell Rezeptors bindet, die für den Subset 2 oder den Subset 4 charakteristisch sind.

3. Biologisches Bindemolekül nach Anspruch 2, **dadurch gekennzeichnet, dass** es an einen Bereich des B-Zell Rezeptors bindet, der durch das Vorhandensein einer Aminosäuresequenz charakterisiert ist, welche ausgewählt ist aus der Gruppe bestehend aus
(i) KLTVLRQPKA (SEQ ID NO. 1),
(ii) VAPGKTAR (SEQ ID NO. 2),
(iii) PTIRRYYYYG (SEQ ID NO. 3), und
(iv) NHKPSNTKV (SEQ ID NO. 4).

4. Biologisches Bindemolekül nach einem der vorhergehenden Ansprüche, **dadurch gekennzeichnet, dass** es ein Antikörper oder ein funktionelles Fragment desselben ist.

5. Biologisches Bindemolekül nach einem der vorhergehenden Ansprüche, **dadurch gekennzeichnet, dass** es in Form eines Fusionsproteins mit T-Zell spezifischen Aktivierungsdomänen vorliegt

6. Biologisches Bindemolekül nach einem der vorhergehenden Ansprüche, **dadurch gekennzeichnet, dass** es mindestens einen zusätzlichen Bereich zum Isolieren oder Abtöten von B-Zell Neoplasien umfasst.

7. Biologisches Bindemolekül nach einem der vorhergehenden Ansprüche, **dadurch gekennzeichnet, dass** es nicht an Rezeptoren oder andere Membranstrukturen von B-Zellen bindet, die keine strukturelle Domäne oder kein Epitop aufweisen, die für den autonom aktiven oder autonom aktivierten Zustand der B-Zell Rezeptoren ursächlich sind.

8. Biologisches Bindemolekül nach Anspruch 7, **dadurch gekennzeichnet, dass** es nicht an Zielsequenzen des B-Zell Rezeptors bindet, die für den Subset 2 oder den Subset 4 nicht charakteristisch sind.

9. Biologisches Bindemolekül nach Anspruch 8, **dadurch gekennzeichnet, dass** es nicht an einen B-Zell Rezeptor bindet, der keine der Sequenzen gemäß Darlegung in Anspruch 3 aufweist.

10. Stoffzusammensetzung, welche ein biologisches Bindemolekül gemäß Definition in einem der Ansprüche 1 bis 9 für prophylaktische und/oder therapeutische Zwecke umfasst.

11. Stoffzusammensetzung nach Anspruch 10, **dadurch gekennzeichnet, dass** sie zusätzlich einen Antikörper oder ein funktionelles Fragment desselben gegen die leichte und/oder schwere Kette des B-Zell Rezeptors umfasst.

12. Verfahren zur Herstellung eines biologischen Bindemoleküls gemäß Definition in einem der Ansprüche 1 bis 9 in üblicher Weise, **dadurch gekennzeichnet, dass** das Bindemolekül durch Immunisieren mit einem IgG Molekül, welches die Aminosäuresequenzen des variablen Teils der schweren Kette des Subset 2 (SEQ ID NO. 5) und der leichten Kette des Subset 2 (SEQ ID NO. 6) aufweist, und anschließende Herstellung von Hybridomazellen gewonnen wird.

13. Verfahren zur Herstellung eines biologischen Bindemoleküls gemäß Definition in einem der Ansprüche 1 bis 9 in üblicher Weise, **dadurch gekennzeichnet, dass** das Bindemolekül durch Immunisieren mit einem IgG Molekül, welches die Aminosäuresequenzen des variablen Teils der schweren Kette des Subset 4 (SEQ ID NO. 7) und der leichten Kette des Subset 4 (SEQ ID NO. 8) aufweist, und anschließende Herstellung von Hybridomazellen gewonnen wird.

14. Verfahren nach Anspruch 12 oder 13, **dadurch gekennzeichnet, dass** das Bindemolekül ein Antikörper oder ein funktionelles Fragment desselben ist.

15. Verwendung des biologischen Bindemoleküls gemäß Definition in einem der Ansprüche 1 bis 9, oder der Stoffzusammensetzung gemäß Definition in Anspruch 10 oder 11, für prophylaktische und/oder therapeutische Zwecke.

16. Verwendung nach Anspruch 15 zur Prophylaxe und/oder Therapie von malignen B-Zell Neoplasien.

17. Verwendung nach Anspruch 16 im Rahmen einer Apharese oder CAR-T-Zell Immuntherapie.
